(19) Europäisches Patentamt / European Patent Office / Office européen des brevets

(11) EP 4 524 141 A1

(12) EUROPEAN PATENT APPLICATION

(43) Date of publication:
19.03.2025 Bulletin 2025/12

(51) International Patent Classification (IPC):
C07D 493/06 (2006.01)

(21) Application number: 24199711.3

(22) Date of filing: 11.09.2024

(52) Cooperative Patent Classification (CPC):
C07D 493/06

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 15.09.2023 LU 505114
18.10.2023 LU 505316

(71) Applicant: Kemijski Institut
1000 Ljubljana (SI)

(72) Inventors:
• LIKOZAR, Blaz
4000 Kranj (SI)
• CAJNKO, Misa Mojca
1000 Ljubljana (SI)
• KOMAR, Jaka
1260 Ljubljana-Polje (SI)

(74) Representative: Weickmann & Weickmann
PartmbB
Postfach 860 820
81635 München (DE)

(54) **SYNTHESIS OF ELLAGIC ACID FROM ETHYL GALLATE USING HYDROXIDES AND DEEP EUTECTIC SOLVENTS**

(57) The present invention provides a method for directly synthetizing ellagic acid from a gallic acid ester, ethyl gallate (EtGal) in hydroxide solution. Product yields and purity can be increased by varying reaction parameters and by adding deep eutectic solvents to the hydroxide solution.

**Fig. 1**

EP 4 524 141 A1

## Description

### Introduction

People are becoming increasingly aware of the importance of maintaining a healthy lifestyle and the role natural plant products play in it. One such valuable natural product is a polyphenolic compound named ellagic acid (EK). EK is a polyphenolic compound found in various fruits and vegetables and other plant sources and has many health-promoting properties like anti-oxidant,[1,2] anti-mutagenic,[3] anti-carcinogenic,[2,4-6] anti-inflammatory,[7] and anti-microbial properties.[8]

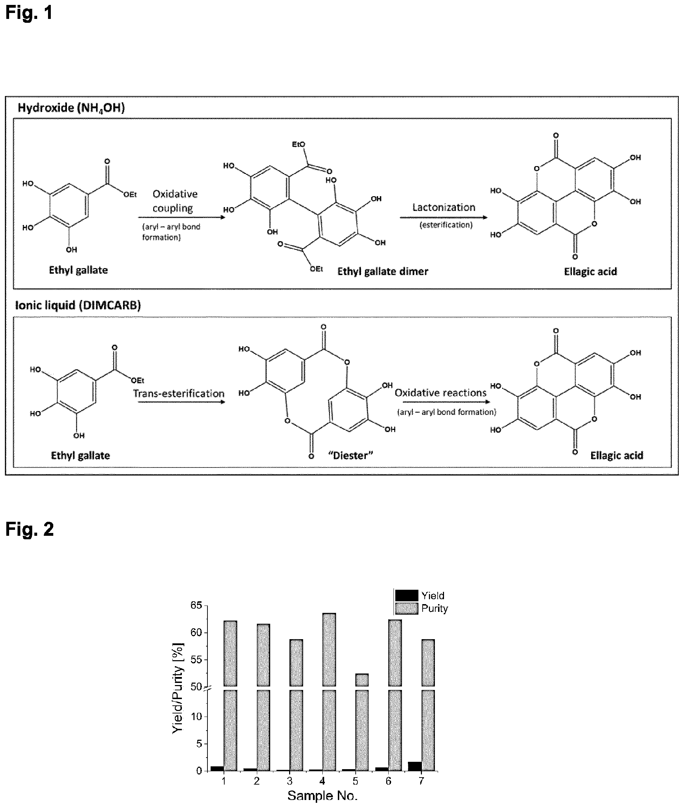

EK is a dimeric derivative of gallic acid. Most of EK is found in a form of hydrolysable tannins called ellagitannins, which are esters of hexahydroxy-dyphenic and glucose.[9-12] EK is mostly produced by hydrolysis of ellagitannins,[13-15] Ellagitannins are first extracted from sources like waste pomegranate husk and then hydrolysed using different chemical methods,[14] fungal fermentation[16] or isolated enzymes.[15] EK can also be directly synthesized from gallic acid or its esters.[17,18] This approach is favourable for industries where no usable by-product is produced and the extraction of EK would come at an expense of other valuable products from the same biomass source that the industry is already utilizing, as well as for the production of EK derivates (symmetric and asymmetric) with increased activity,[19,20] There are a few different reaction mechanisms of direct EK synthesis found in literature (Fig. 1). Chowdhury *et al.*[18] proposed a mechanism of EK synthesis from ethyl gallate (EtGal) in an ionic liquid that involves trans-esterification of two EtGal molecules and subsequent oxidative reactions to produce the intramolecular C-C bond (aryl-aryl bond) and thus, EK. Others described different varieties of oxidative coupling where esterified gallic acid is first dimerized through aryl-aryl bond formation, followed by formation of the lactone ring (esterification).[17,20-22] One group even described a mechanism where EK and its derivate were synthetized from gallic acid ester through ellagitannin analogue and its subsequent hydrolysis.[19]

### Summary of the Invention

The study underlying the present invention aimed at directly synthetizing EK from a gallic acid ester, ethyl gallate (EtGal). Simple reaction protocols from literature were used, one with an ionic liquid[18] and another with a hydroxide and aeration.[17] In the present invention, EK yields and purity could be increased by varying reaction parameters and by adding deep eutectic solvents to the hydroxide solution. An ionic liquid N,N-dimethylammonium N',N'-dimethylcarbamate (DIMCARB), 3 different hydroxides and aeration and 5 choline chloride (ChCl)-based deep eutectic solvents (DES) in combination with a hydroxide and aeration were used. Despite optimization attempts, DIMCARB performed very poorly (< 2% yield of EK). On the other hand, the reactions with 1 % $NH_4OH$ catalyst resulted in a 50.3 % yield of EK with 85 % purity. When 10 % DES were added to 1 % $NH_4OH$, the yields increased up to 77.5 % with 82.9 % purity.

The reactions with the highest yields were also tested in a simple scale-up process. A scale-up of the most efficient system (DES/$NH_4OH$) resulted in yields reaching 61.8 % and purity over 80 %, respectively, indicating importance of aeration. A kinetic model was developed linking the experimental results and the reaction conditions, providing additional insights into the process mechanisms. A model of the synthesis process showed that pH is the most important factor influencing the reaction rate and the solubility of the product. It was confirmed that DES reduces the solubility of ellagic acid, which partly explains the drastic increase in yield.

### Detailed description

The present invention provides a method for preparing ellagic acid directly from ethyl gallate, comprising the steps

(i) reacting ethyl gallate with a hydroxide compound in the presence of air and a deep eutectic solvent (DES) at alkaline pH, and

(ii) isolating ellagic acid.

The hydroxide compound acts as a reaction catalyst and supports oxidative coupling and esterification to yield ellagic acid directly from ethyl gallate. It can for example be selected from ammonium hydroxide $NH_4OH$, alkali metal hydroxides such as NaOH and KOH, and mixtures thereof. In particularly preferred embodiments, the hydroxide compound is $NH_4OH$.

The concentration of the hydroxide compound, in particular $NH_4OH$, in the reaction mixture can be in a range of 0.5-5 % based on the total weight of the reaction mixture. Preferably, the concentration of the hydroxide compound is in a range of 0.8-1.2 %, more preferably about 1 %.

The pH of the reaction mixture affects the reaction. According to the invention, an alkaline pH is essential to obtain the desired reaction product. Preferably, the pH of the reaction mixture is about 9-13, more preferably 10-12, in particular about 11.

Deep eutectic solvents are mixtures of two or more pure compounds (mostly in solid form), a hydrogen bond donor and a hydrogen bond acceptor. When combined at specific molar ratios, they form a liquid that exhibits an eutectic point temperature that is far below an ideal liquid mixture and present low vapor pressure and high thermal stability.[37] DES have been shown to modulate (increase) compound solubility, thermal stability and reaction efficiency.[38-41] These solvents have also been used in (trans)esterification reactions as sole catalysts or in combination with hydroxides.[24,25,27,42,43] In the invention, it was found that adding a DES improves the yield and purity of the desired ellagic acid product. The DES is selected to have an alkaline pH.

In particular embodiments of the invention, the DES comprises 5-choline chloride (ChCl) in combination with at least one additional solvent such as glycerol and urea. Preferably, the ration of ChCl to the additional solvent(s) (in particular ChCl:glycerol or ChCl:urea) is about 1:1 to 1:3, more preferably about 1:2.

The concentration of the DES in the reaction mixture can be in a range of 5-20 % based on the total weight of the reaction mixture. Ppreferably, the concentration of the DES is in a range of 8-12 %, more preferably about 10%.

The mixture to be reacted in step (i) can be obtained by preparing a solution comprising the hydroxide compound and the DES and adding ethyl gallate to the solution to yield a reaction mixture at alkaline pH.

The reaction time in step (i) can be about 24-72 h, preferably 42-54 h, more preferably about 48 h.

The reaction in step (i) is carried out at room temperature.

The presence of air in step (i) is required for oxidative coupling of ethyl gallate. It can be promoted by air bubbling, i.e. by bubbling air though the reaction mixture. When air bubbling is introduced, the yields of the desired product increase. Preferably, the reaction in step (i) is carried out with stirring.

The invention is further illustrated by the following Figures and Examples.

**Figures**

**Fig. 1** Proposed mechanism of ellagic acid synthesis from ethyl gallate with hydroxides[17] or ionic liquids.[18]

**Fig. 2** Yield and purity of 7 different ellagic acid samples synthetized with DIMCARB

**Fig. 3** Yield and purity of ellagic acid samples synthetized with 1 % NH4OH at different reaction times (a) or different concentrations of NH4OH (b), KOH (b) and NaOH (c) after 48 h.

**Fig. 4** Yield and purity of ellagic acid samples synthetized with different concentrations of DES1 (a) or DES2 (b) in 1 % after 48 h. DES1 - ChCl:glycerol 1:2; DES2 - ChCl:urea 1:2.

**Fig. 5** SEM images of ellagic acid standard and ellagic acid samples synthetized with DIMCARB, 1 % NH4OH and 10 % DES1 in 1 % NH4OH. EK - ellagic acid; DES1 - ChCl:glycerol 1:2.

**Fig. 6** Comparison of model and experimental data of pure ellagic acid precipitate during the variation of a) time at 1 % wt. NH4OH without DES and b) NH4OH initial concentration measured at 48 h without DES.

**Fig. 7** The introduction of DES1 in 1% wt. $NH_4OH$ may increase the product yield (after 48 hours) by reducing its solubility. However, this cannot explain the experimental trends. Grey - no effect on solubility, red - solubility of EK is reduced 1000-fold, black

**Fig. 8** Scale-up process: yield and purity of ellagic acid product from reactions with 10 % DES1 (a) or 10 % DES2 (b) in 1 % NH4OH with or without air bubbling. DES1 - ChCl:glycerol 1:2; DES2 - ChCl:urea 1:2.

**Fig. 9** Chromatogram and UV-VIS spectra of ellagic acid standard

**Fig. 10** Chromatogram and UV-VIS spectra of ethyl gallate standard

**Fig. 11** Chromatogram and UV-VIS spectra of ellagic acid synthesized with DIMCARB

**Fig. 12** Chromatogram and UV-VIS spectra of ellagic acid synthesized with 1 % NH4OH

**Fig. 13** Chromatogram and UV-VIS spectra of ellagic acid synthesized with 1 % KOH

**Fig. 14** Chromatogram and UV-VIS spectra of ellagic acid synthesized with 1 % NaOH

**Fig. 15** Chromatogram and UV-VIS spectra of ellagic acid synthesized with 10 % DES1 in 1 % $NH_4OH$

**Fig. 16** Chromatogram and UV-VIS spectra of ellagic acid synthesized with 10 % DES1 in 1 % $NH_4OH$

**Fig. 17** Ellagic acid in the form of crystals after synthesis in DIMCARB (a) and in the form of light brown powder after synthesis with 10 % DES1 in 1 % $NH_4OH$ (b)

**Fig. 18** Ellagic acid control reaction: Yield and purity of ellagic acid incubated in 1 % $NH_4OH$ and 10 % DES1 in 1 % $NH_4OH$ reaction media.

**Fig. 19** SEM images of ellagic acid crystals synthetized with DIMCARB at 20000X magnification

**Fig. 20** Compiled data about ellagic acid solubility in the dependence of temperature and pH. Solubility increases consistently at two distinct pH values, namely around 5.6 and around 8.5, which is not temperature dependent. The black dotted line based on Henderson-Hasselbach equation is the one used in simulated, calibrated to our solubility experiment. The coefficients can be found in Table 3 below.

Henderson-Hasselbach equation:

$$S = S0 \cdot (1 + 10^{pH-pKa})$$

**Table 3** *Henderson-Hasselbach coefficients at different conditions of ellagic acid*

| conditions | 25 °C-basic | 90 °C-acid to neutral |
|---|---|---|
| **S0 [mg/L]** | 3 | 30 |
| **pKa** | 8.50 | 5.60 |

**Fig. 21** SEM-EDS mapping of $NH_4OH$-DES1 sample.

It can be found that N is evenly distributed, supporting the claim that final product is ammonium salt.

**Examples**

**Experimental**

*Materials*

Ellagic acid (EK; Thermo Scientific), Ethyl gallate (EtGal; Thermo Scientific), N,N-dimethylammonium N',N'-dimethylcarbamate (DIMCARB; Acros Organics); $NH_4OH$ (Roth), KOH (Honneywell); NaOH (Supelco); Choline chloride (ChCl; Sigma-Aldrich); Glycerol (Sigma-Aldrich); Urea (Acros Organics); p-toluene sulfonic acid (p-TSA; Fluka); Acetic acid (Emsure); Oxalic acid (Sigma-Aldrich).

*Synthesis of EK with ionic liquids*

The synthesis of EK using ionic liquids was carried out based on the method by Chowdhury *et al*..[18] The ionic liquid used was DIMCARB. In the initial experiment, 660 mg EtGal was dissolved in 4.43 g DIMCARB and 0.12 g water and left for 6 h at room temperature (RT) and 150 rpm using magnetic stirrer. Afterwards 2.3 g of water was added to the mixture and left for EK to precipitate overnight. The protocol was then modified by using either 330 or 660 mg EtGal, reaction times 6 or 9 h, addition of 2.3 (1x), 4.6 (2x) or 6.8 (3x) g of water and precipitation times up to 14 days. After the reaction, the mixture was transferred to a 14 mL centrifuge tube and centrifuged at 6800 rcf for 10 minutes. The supernatant was removed and the pellet thoroughly mixed (washed) with 14 mL of lukewarm water and centrifuged again. This washing procedure was repeated 6 times. After the final washing and centrifugation, the remaining pellet was dried for 24 h at 75°C.

*Synthesis of EK using hydroxides*

The initial protocol for synthesis of EK in $NH_4OH$ with aeration was based on the method by Zeng *et al*.,[17] and other hydroxides, KOH and NaOH, were selected based on literature where they were used in (trans)esterification reactions.[23-26] A typical reaction consisted of 200 mg of EtGal dissolved in 4 mL of hydroxide solution in a glass bottle with cap and left at RT and 150 rpm. The cap was not screwed on tight to let in air. Afterwards, the mixture was transferred to a 14 mL centrifuge tube and centrifuged at 6800 rcf for 10 minutes. The supernatant was removed and the pellet thoroughly mixed (washed) with 14 mL of lukewarm water and centrifuged again. This washing procedure was repeated 6 times. After the final washing and centrifugation, the remaining pellet was dried for 24 h at 75°C. First 1 (v/v) % of $NH_4OH$ was used in 24, 48 and 72 h reactions to determine the optimal reaction time. Then, 0.5, 1, 3 and 5 (v/v) % $NH_4OH$, KOH and NaOH were tested in 48 h reactions. The pH values of the hydroxide solutions are presented in Table 1. A control reaction was also prepared where EK instead of EtGal was mixed with 1 % $NH_4OH$ and left for 48 h at room temperature. Standard deviation was calculated based on 2 - 4 separate experiments.

*Synthesis of EK using deep eutectic solvents and $NH_4OH$*

The DES used were selected based on literature where they were used in esterification and transesterification reactions.[24,25,27] The selected DES were: ChCl:glycerol 1:2 (DES1), ChCl:urea 1:2 (DES2), ChCl:p-TSA 1:3 (DES3), ChCl:acetic acid 1:2 (DES4) and ChCl:oxalic acid 1:2 (DES5). The DES were used in 5, 10 and 20 (w/v) % concentrations and were diluted by either water or 1 % $NH_4OH$. A typical reaction mixture for 10 % DES consisted of 200 mg EtGal, 400 mg of DES and 3.6 mL of 1 % $NH_4OH$ or water. The mixture of DES and $NH_4OH$ or water was prepared first and mixed until a homogenous liquid was formed, and afterwards EtGal was added. The reaction time was 48 h and all other reaction and preparation steps were the same as for reactions with hydroxides. The pH values for all of the DES/NH$_4$OH solutions are presented in Table 1. A control reaction was also prepared where EK instead of EtGal was mixed with 10 % DES1 in 1 % NH$_4$OH and left for 48 h at room temperature. Standard deviation was calculated based on at 2 - 4 separate experiments.

**Table 1** *pH values of reaction solutions without ethyl gallate*

| Solution | [%] | pH | Solution | [%] | pH | Solution | [%] | pH |
|---|---|---|---|---|---|---|---|---|
| **NaOH** | 0.5 | 13.2 | **KOH** | 0.5 | 12.9 | **NH$_4$OH** | 0.5 | 11.3 |
| | 1 | 13.5 | | 1 | 13.2 | | 1 | 11.4 |
| | 3 | 13.8 | | 3 | 13.7 | | 3 | 11.8 |
| | 5 | 13.8 | | 5 | 13.9 | | 5 | 12.0 |
| **DES1 in NH$_4$OH** | 5 | 11.4 | **DES2 in NH$_4$OH** | 5 | 11.4 | **DES3 in NH$_4$OH** | 10 | 2.3 |
| | 10 | 11.3 | | 10 | 11.3 | **DES4 in NH$_4$OH** | 10 | 4.7 |
| | 20 | 11.2 | | 20 | 11.1 | **DES5 in NH$_4$OH** | 10 | 3.9 |

*HPLC analysis*

The amount of EK (purity) in the samples was determined using HPLC. Each sample was dissolved in 100 % methanol with 0.1 % formic acid (protonation of the EK OH-groups) to a final concentration of 50 mg/L, sonicated for 3 minutes and filtered through 0.22 μm syringe filter. The samples were then analysed using HPLC system (Dionex Ultimate 3000, Thermo Scientific) with a Kinetex C18 column (5 μm, 100 A, 100×4.6 mm, Sigma Aldrich). The mobile phase consisted of distilled water with 0.1 % formic acid (A) and 100 % methanol with 0.1 % formic acid (B). The column temperature was set to 40°C, flow rate at 1 mL min$^{-1}$, injection volume 10 μL and detection at UV-Vis absorption wavelength 366 nm for EK and 275 nm for

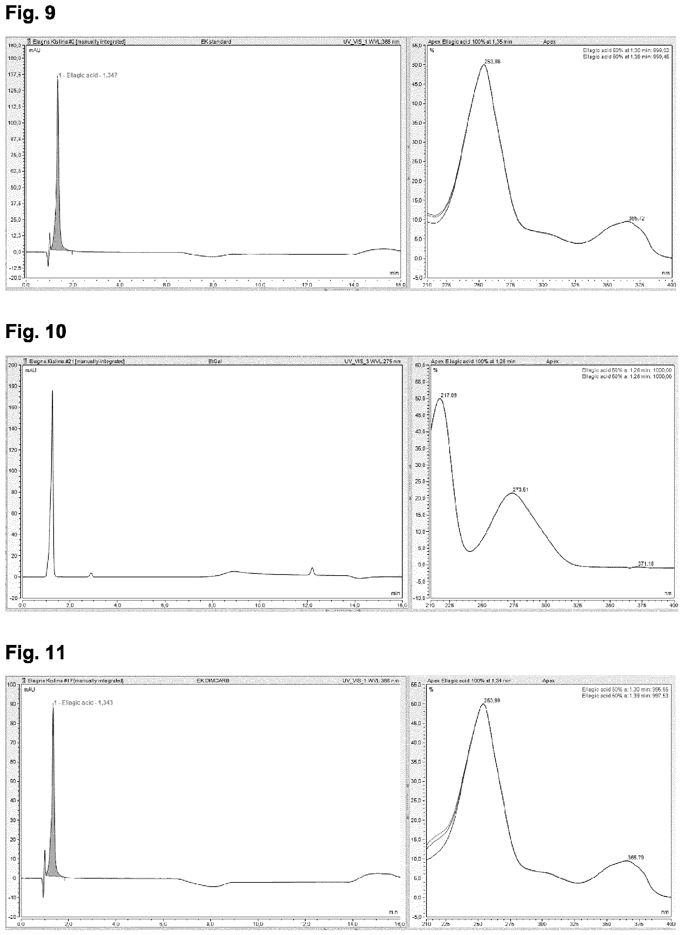

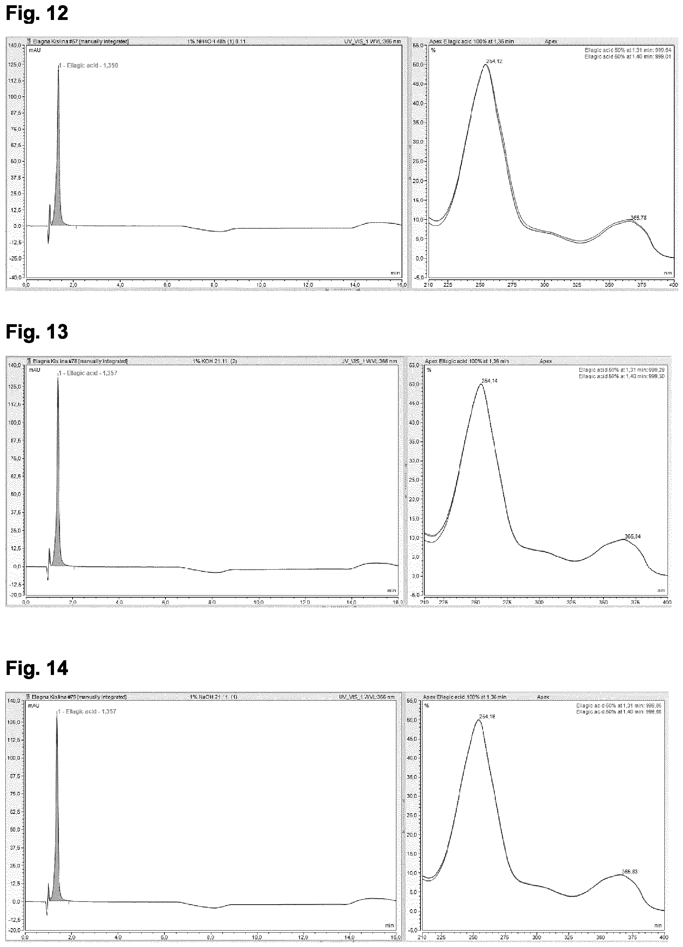

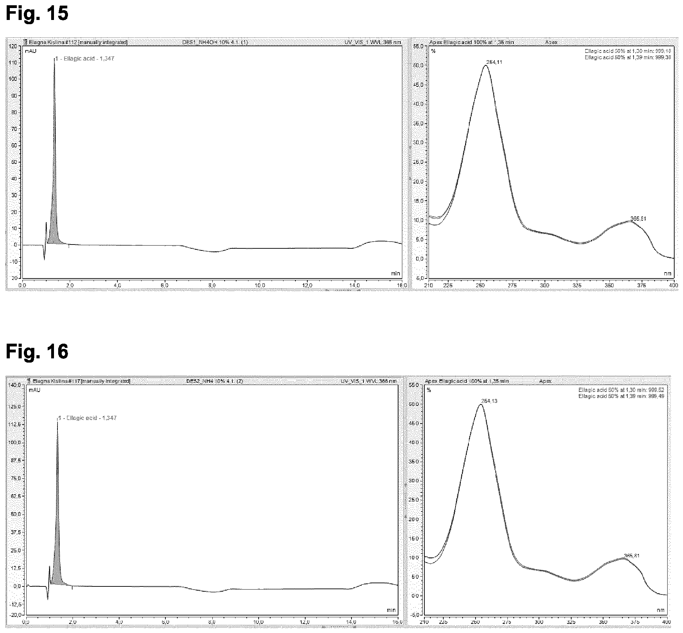

EtGal. The mobile phase gradient was as follows: 0 - 5 min 40 % A and 60 % B, 5 - 7 min 60 - 90 % B, 7 - 12 min 10 % A and 90 % B, 12 - 14 min 90 - 40 % mobile phase B and 14 - 16 min 40 % A and 60 % B. Ellagic acid in the samples was identified and quantified by the external calibration standard (retention time and UV-VIS spectra). The retention times were 1.35 min for EK and 1.17 min for EtGal. The UV-VIS spectra of ellagic acid standard showed signature peaks at 254 nm 367 nm, and EtGal at 217 and 273 nm. The chromatograms and UV-VIS spectra of EK, EtGal and selected samples are presented in Fig. 9-16.

*Scanning electron microscopy and elemental analysis*

The surface morphology of the samples was analysed by scanning electron microscopy coupled with energy dispersive spectroscopy (SEM; SUPRA35 VP, Carl Zeiss, EDS: SDD Ultim max 100, Oxford Laica). The samples were mounted on aluminium stubs with double-sided carbon tape and sputtered with a 2.3 nm thick Au coating before observation to ensure sufficient conductivity. SEM-EDS was performed at 20 kV and at three different locations. SEM-EDS mapping was also performed to obtain the distribution of impurities.

*Scale-up*

The scale-up experiment was carried out only for 10 % DES1 and DES2 in 1 % NH4OH reactions. The reaction mixtures were prepared in 500 mL beakers and consisted of 10 g EtGal, 20 g of selected DES and 180 mL of 1 % NH$_4$OH. The mixture of DES and NH$_4$OH was prepared first and mixed until homogenous liquid was formed, and afterwards EtGal was added. In the first experiment the reaction mixture was only stirred at 150 rpm, and in the second, we also introduced air bubbling into the solution. The mixtures were left for 48 h and the resulting product centrifuged and washed 6 times in lukewarm water and dried at 75°C. Each experiment was performed only one time with one parallel.

**Theoretical**

Since a large influence of pH on product yield was observed, hydroxyl groups were included as reaction catalyst in the kinetic modelling. Transesterification can occur under basic conditions,[28] while the biphenyl bond is typically formed by an acid catalyst and an oxidant.[29] For this reason, we have chosen the first or second transesterification in the gallate dimer molecule as the rate-determining step (Eq.1) (the kinetic expression is the same in both cases). We assume that the concentrations in the reactor are uniform. The by-products are not part of the model.

$$2\frac{d[EK]}{dt} = k_f[EtGal]^2[OH^-] - k_b[EK][OH^-] \qquad \text{(Eq. 1)}$$

Here $k_f$ and and $k_b$ stand for the lumped kinetic coefficients of the forward and reverse reactions and [OH -] for the molar concentration of the hydroxyl groups in the solution, which correlates with a NH3 pKb of 4.75. The pKa of the deprotonation of the phenolic hydroxyl groups present in EtGal are 8.7, 11.4 and above 13[30], resulting in an initial pH decrease. For simplicity of the model, we have assumed single deprotonation for EtGal and double deprotonation for ellagic acid, resulting in a constant pH during the reaction. The deprotonation of ellagic acid is explained below. Therefore, the initial pH was corrected for the addition of EtGal by subtracting the initial $NH_4OH$ concentration.

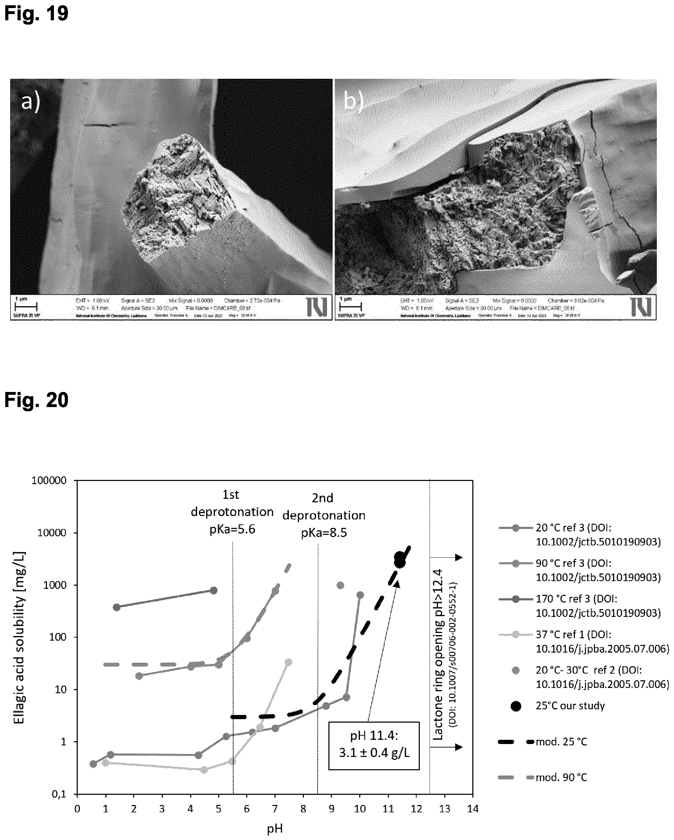

Since ellagic acid is poorly soluble in water, it precipitates during the reaction. However, the solubility is pH-dependent, with deprotonation of the phenolic hydroxyl groups leading to increased solubility under basic conditions. Literature indicates that ellagic acid undergoes at least 2 stages of deprotonation (at pH 5.6 and 8.5[31]), with solubility increasing with increasing pH. At pH 12.4 and above, hydrolysis of the lactone moiety was observed, resulting in a carboxyl derivative.[32] We have compiled all available data on the solubility of ellagic acid in the range of pH 0.6 - 10 and temperatures between 20°C - 170°C from various sources[31-34] from which trends can be derived that correlate with the Henderson-Hasselbach equation, as in the work of Bala et al.[34] (Figure 20). However, the typical pH used here is 11.4, which was measured separately in our work (3.1±0.4 g/L) using 40 mg ellagic acid in 0.5 mL5 wt% $NH_4OH$ (1 h at 25°C), centrifugation and precipitation with 10% HCOOH to achieve a pH of 3, followed by centrifugation and drying of the precipitate at 75 °C overnight. The Henderson-Hasselbach coefficients of the 2nd deprotonation of ellagic acid to determine solubility were estimated for 25°C (pKa = 8.5, S0 = 3mg/L) (Table 3). The [EK], the ellagic acid concentration, was limited by the solubility, which was determined with the following equation:

$$S(EK) = S0 \cdot (10^{pH-pKa} + 1) \qquad \text{(Eq. 2)}$$

**Results and discussion**

*Synthesis of EK with DIMCARB*

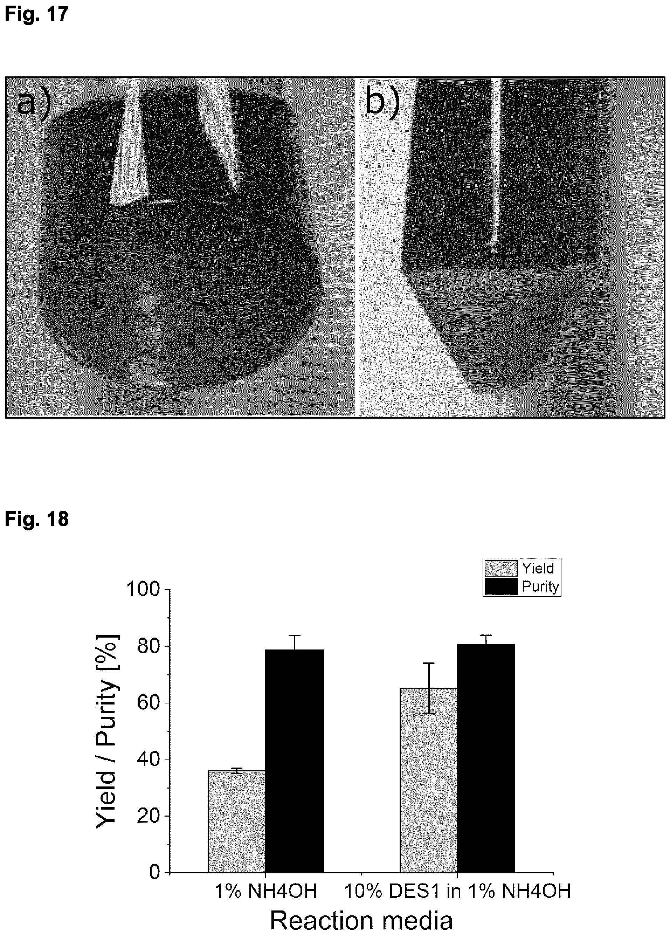

Our first attempt at EK synthesis was based on a study by Chowdhury *et al.*[18] where they used ionic liquid DIMCARB as solvent/catalyst to produce EK from EtGal. We used the original protocol as well as adapted versions with various amounts of EtGal, reaction and precipitation times and amounts of added water for EK product precipitation. Although Chowdhury *et al.* [18] reported a 70 % EK yield, we obtained no visible product when using their original protocol. Only after the reaction time was extended from 6 to 9 h, the amount of water increased from 1x to 3x and precipitation time extended to more than 24 h, did we obtain some EK product in a form of light-yellow crystals (Fig. 17a). However, despite these protocol adaptations, the highest yield of EK was only 1.7 % with 29.4 % purity. The yields were also very variable, ranging from the maximum 1.7 % to as low as 0.2 % and purity from 26.2 to 31.8 % (Fig. 2). Due to these low and variable yields, the synthesis of EK with DIMCARB was not pursued further.

*Synthesis of EK with hydroxides*

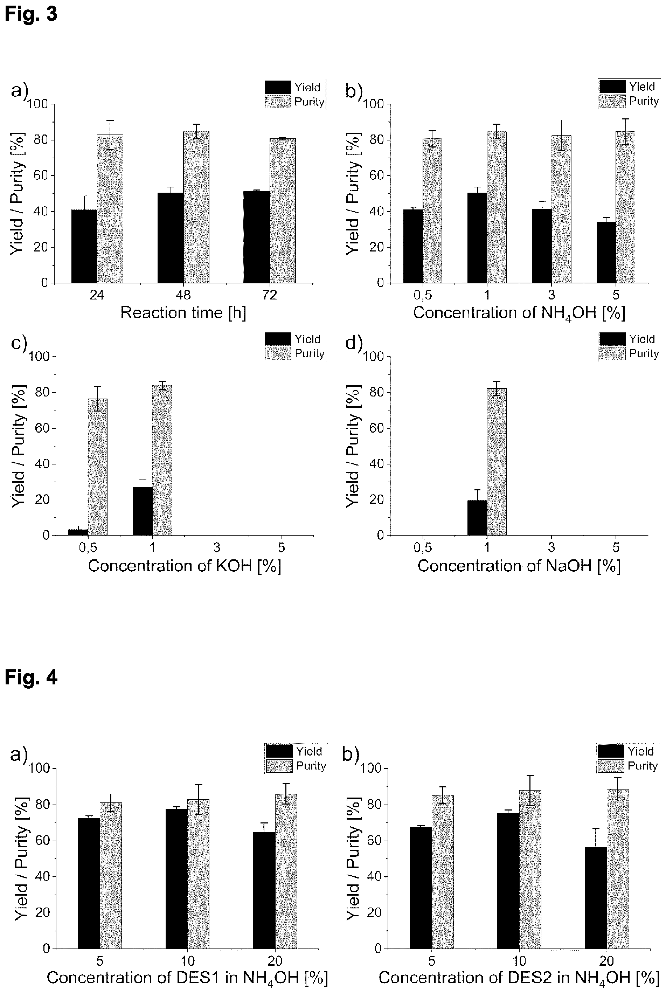

Based on the proposed reaction mechanisms,[17,20-22] the synthesis of EK from EtGal involves oxidation reactions (oxidative coupling) and esterification (lactonization) of two EtGal molecules. The oxidant in these reactions is provided by air (oxygen) and the catalyst for esterification is a hydroxide. Hydroxides like KOH and NaOH have previously been used to catalyze these types of reactions,[23-26] however, we first focused on $NH_4OH$ since it has already been used in a similar EK synthesis reaction with good results.[17] Reactions of EtGal in 1 % $NH_4OH$ were prepared and left for 24, 48 and 72 h in order to determine the best reaction times. As shown in Fig. 3a, EK yield after 24 h was 41.1 % and the yields after 48 h and 72 h were 50.3 and 51.5 %, respectively. The purity of all of the EK products was between 80 and 85 %, with the highest being that of 48 h samples (84.7 %). Since the difference in EK yields between 48 and 72 h reaction time was minimal and the purity of 48 h samples seemed to be the highest, 48 h reaction time was used in all further reactions. In the next step, different concentrations (0.5, 1, 3 and 5 %) of $NH_4OH$ as well as KOH and NaOH were tested (Fig. 3b-d). The results in Fig. 3b show, that the most optimal concentration of $NH_4OH$ was 1 % with 50.3 % yield, and the least optimal 5 % with 33.9 % yield. The purity of all samples was again between 80 and 85 %. In reactions with KOH, a product was obtained only in 0.5 and 1 % solution with 3.2 and 27.8 % yield and 76.6 and 83.9 % purity, respectively (Fig. 3c). The yields were the lowest with NaOH, where we obtained a 19.5 % yield (82.2 % purity) with 1 % solution and no product with other concentrations (Fig. 3d). The low yield when using KOH and NaOH could be explained by the large pH change when EtGal is introduced into the solution. As mentioned earlier in the Theoretical section, the phenolic hydroxide of gallic acid deprotonates at about 8.7[31], which means that almost all of the hydroxide is neutralised at a concentration of 0.5% and 1% NaOH and KOH, resulting in low conversion activity. At concentrations of 3% and more, the pH value on addition is above 12.4, favouring lactone ring opening (also taking into account the neutralisation of the second proton at pKb=11.4). No EtGal was detected in any of the samples and all of the samples were in a form of a fine light brown powder (Fig. 17b). When EK instead of EtGal was used in the reaction with 1 % $NH_4OH$ (control), the resulting product was green in colour with a yield and purity of 36.1 and 78.8 %, respectively (Fig. 18).

The research on direct synthesis of EK or its derivates from gallic acid or its esters is relatively scarce. Researchers used different one-step methods like direct oxidative coupling with hydroxides or oxidants[17,22] and enzymatic coupling,[17] or multi-step methods involving Ullman coupling[21] or a combination of oxidants and esterifying agents (methanol).[22] An 83 % yield of EK was obtained in 24 h using a one-step reaction of methyl gallate and the oxidant phenyliodine bis(trifluoroacetate) (PIFA).[22] The same group also used a two-step reaction. There, methyl gallate was first dimerized by oxidative coupling in the presence of the oxidant phenyliodine diacetate (PIDA) yielding 80 % of the biaryl in 16 h, which was then quantitatively converted to EK by refluxing with aqueous methanol in 24 h. Our method using hydroxides was based on the method by Zeng *et al.*[17] where they used methyl gallate and $NH_4OH$ and aeration to produce EK with a yield of 47 % and 88 % purity in 24 h. When they used a tyrosinase enzyme instead of $NH_4OH$, the yield and purity increased to 62 % and > 98 %, respectively. Our results, when using $NH_4OH$/aeration protocol, were in good agreement with this study, with 50.3 % yield and 84.7 % purity of the EK product (Fig. 3b).

Compared to $NH_4OH$, the other two hydroxides performed very poorly. Research has shown that EK is strongly affected by pH, and strongly alkaline pH results in spontaneous lactone ring-opening in the EK molecule.[32,34,35] Therefore, we considered the pH of the solution as one of the factors influencing the efficiency of the reaction. Table 1 shows that all hydroxide solutions had a strongly alkaline pH (11.3 - 13.9). Nonetheless, EK synthesis was still very efficient in 1 % $NH_4OH$ despite the 11.3 pH. However, EK yield was reduced at higher pH values measured for 3 and 5 % $NH_4OH$ as well as for all KOH and NaOH solutions. Hasegawa *et al.*[32] showed that the lactone ring opening occurs at pH value 12.4. This supports our results that show that even 5 % $NH_4OH$ with pH 12.0 resulted in a higher yield of EK than the lowest concentrations of KOH or NaOH with pH 12.9 and 13.2, respectively. On the other hand, the pH of 0.5 % $NH_4OH$ was slightly lower than 1 % solution, but resulted in lower EK yields, thus, it seems that in this case, the amount of hydroxide catalyst was also a limiting factor.

Considering the impurities, the bulk of them are probably reaction intermediates and unspecific products like EK with an open lactone ring, dimers, condensed structures, flavellagic acid and quinones.[17,32,34,36] However, when analysed by HPLC, no other peaks were detected. This is either because these products did not bind to the HPLC column or did not absorb at the selected wavelengths. Deprotonated forms of EK also would not be detected since the sample was dissolved in acidified methanol prior to analysis, which would protonate the OH-groups.

When the same amount of EK instead of EtGal was used in the reaction with 1 % $NH_4OH$, the final product was a green colour instead of light brown, and the yield (the remaining EK in the solution) was significantly lower than 100 %. A part of the EK was surely lost during the washing process and some of it was dissolved in the reaction solution, but these losses should be minimal. More likely, EK reacted with the $NH_4OH$ catalyst, which affected its solubility. This reaction between EK and $NH_4OH$ is also supported by the reduced purity of this EK. The fact that the reaction with EtGal resulted in higher EK yields compared to the control indicates that reaction by-products like ethanol could play a role in preventing the interaction between the produced EK and the $NH_4OH$ catalyst. Also, the similar purity of the synthetized EK and EK control, points to EK-$NH_4OH$ interaction products comprising the majority of the impurities.

*Synthesis of EK in DES mixtures*

Hydroxides have previously been successfully used in combination with DES in (trans)esterification reactions.[24,25,27] We prepared 5 different ChCl-based DES and diluted them to a final concentration of 5, 10 or 20 % by either 1 % $NH_4OH$ or water. Reactions of EtGal in DES/$NH_4OH$ or DES/water were prepared and incubated for 48 h. The reaction conditions and subsequent product preparation were identical to those with only 1 % $NH_4OH$ and other hydroxides. An EK product was formed only with DES1 (ChCl:glycerol 1:2) and DES2 (ChCl:urea 1:2) in combination with 1 % $NH_4OH$ (Fig. 4). Reactions with DES3 (ChCl:p-TSA 1:3), DES4 (ChCl:acetic acid 1:2) and DES5 (ChCl:oxalic acid 1:2) did not yield a product and neither did reactions with DES1 and 2 in combination with water (data not shown). Compared to reactions with only 1 % $NH_4OH$ (Fig. 3b), the addition of either DES1 (Fig. 4a) or DES2 (Fig. 4b) markedly increased product yields. The highest EK yields were obtained in 10 % DES1 and DES2 mixtures and were 77.5 and 75.1 % with 82.9 and 87.8 % purity, respectively. The other two DES concentrations (5 and 20 %) were less efficient when considering product yields but with similar product purity (between 81.1 and 88.4 %). The lowest yields were obtained with 20 % DES1 and DES2, 64.7 and 56.3 %, respectively. No EtGal was detected in any of the samples. As a control, EK instead of EtGal was used in the reaction with 10 % DES1 in 1 % $NH_4OH$ and the resulting product once more displayed a green colour and a yield and purity of 65.2 % and 80.6 %, respectively (Fig. 18).

Deep eutectic solvents are mixtures of two or more pure compounds (mostly in solid form), a hydrogen bond donor and a hydrogen bond acceptor. When combined at specific molar ratios, they form a liquid that exhibits an eutectic point temperature that is far below an ideal liquid mixture and present low vapor pressure and high thermal stability.[37] DES have been shown to modulate (increase) compound solubility, thermal stability and reaction efficiency.[38-41] These solvents have also been used in (trans)esterification reactions as sole catalysts or in combination with hydroxides.[24,25,27,42,43]

As shown in the previous section, pH seems to affect EK synthesis. This is again evident here since the only DES solutions that produced EK, i.e. DES1 and DES2 mixtures, were the ones with alkaline pH as opposed to the ones with acidic pH (DES3-5). Furthermore, the fact that reactions in DES/water mixtures did not yield any product indicates that the DES were not acting as catalysts but rather as solubility modulators. It is possible that DES3-5 increased the solubility of EK which subsequently did not precipitate from the solution and/or inactivated the alkaline $NH_4OH$ preventing it from catalyzing the reaction. The effectiveness of DES1 and 2 mixtures is most likely connected to reduced solubility of EK. This is supported by the control reaction where EK instead of EtGal was used (Fig. 18). Although the product was still a different colour (green instead of light brown), the yield (remaining EK in solution) in DES1/$NH_4OH$ mixture was 65.2 % compared to only 36.1 % in only $NH_4OH$ solution. However, the purity of the extracted EK was still reduced (80.6 %) and was similar to that of synthetized EK product, which indicates that the interactions between EK and $NH_4OH$ still took place.

*Morphology and elemental analysis*

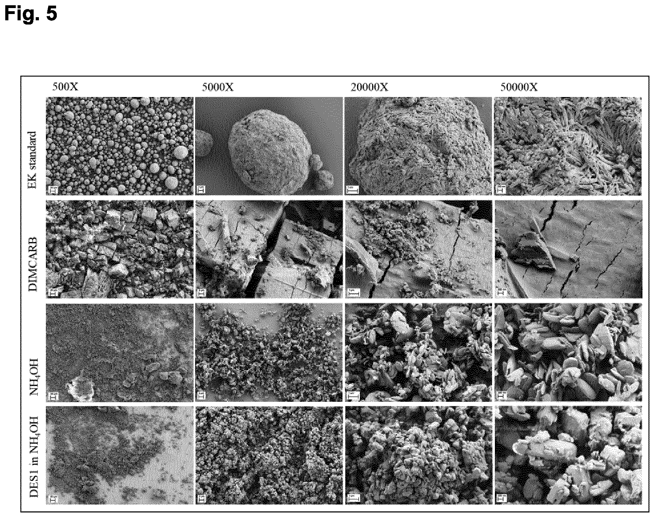

The morphological features of EK standard and DIMCARB, 1 % $NH_4OH$ and 10 % DES1 in 1 % $NH_4OH$ samples were analyzed by SEM and are presented in Fig. 5. The images were taken at 4 different magnifications. All of the samples showed distinct morphological characteristics. EK standard showed spherical shapes of different diameter that seem to be composed of mostly smaller rod-like structures. DIMCARB samples exhibited rectangular formations that originated from larger crystals that could be seen in the reaction mixture (Fig. 17). Based on the cracked surface, the crystals ware likely very brittle and would break apart into much smaller particles. Another interesting feature that was visualized in certain parts of the sample was a kind of smooth crust on the outside of the large particles with small, ordered, rod-shaped formations on the inside (Fig. 19a and b). $NH_4OH$ and DES1 in $NH_4OH$ samples looked similar at 500x magnification, but distinct differences were observed at higher magnifications. $NH_4OH$ sample showed flattened elliptical shapes that came in a wide variety of sizes, whereas the DES1 in $NH_4OH$ sample showed a variety of different shapes like irregular spheres, rectangular shapes and rods, also in a wide variety of sizes. Compared to EK standard, particle size in these samples was significantly smaller.

EDS analysis was also performed on the samples (Table 2). It showed an increased amount of N in EK samples synthetized with DIMCARB, 1 % $NH_4OH$ and 10 % DES in 1 % $NH_4OH$ compared to EK standard. The amount of N in the EK standard was 0.2 wt.%, whereas the amount of N in DIMCARB, 1 % $NH_4OH$ and 10 % DES in 1 % $NH_4OH$ was 6.7, 5.5 and 8 wt. %, respectively. 10 % DES in 1 % $NH_4OH$ also contained 0.5 wt.% of Cl.

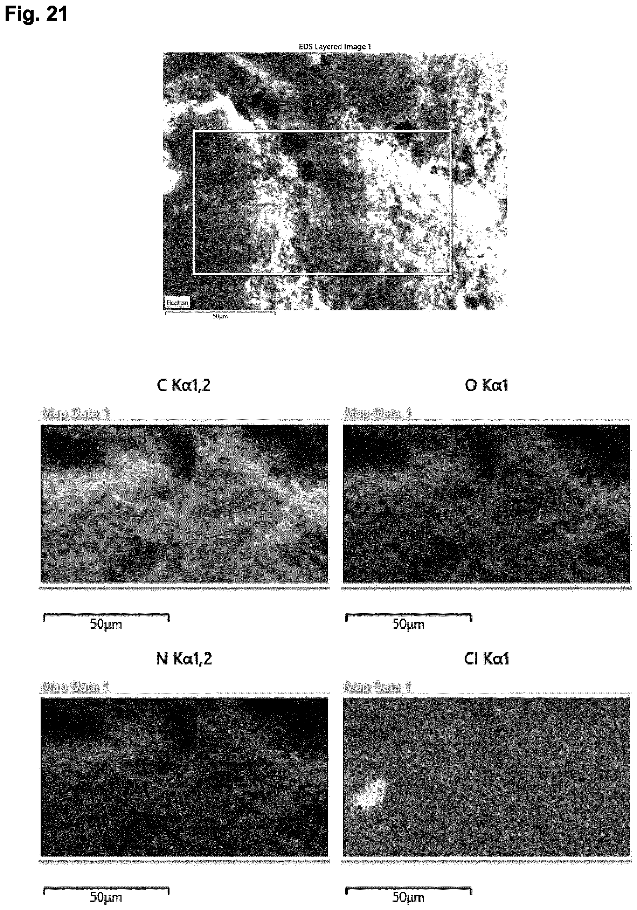

We also observed differences in the O/C and N/C ratios, which could be due to a different particle size of the precipitate, resulting in less decomposition of the ammonium salt during drying or washing. Based on the pKa values, a diammonium ellagate salt is formed first and therefore the theoretical range of nitrogen per ellagic acid ($C_{14}H_6O_8$) is between 0 and 2, which can also be seen in Table 2. Furthermore, the SEM-EDS mapping results support this claim, with N evenly distributed across the sample (Fig. 21). The slightly larger measured O/C ratio than expected (7.4$\pm$0.2, theoretically 8) could be due to the background contribution of the graphite tape.

**Table 2** *EDS analysis of ellagic acid standard and ellagic acid samples synthetized with DIMCARB, 1 % $NH_4OH$ and 10 % DES1 in 1 % $NH_4OH$. EK - ellagic acid; DES1 - ChCl:glycerol 1:2.*

| Name | Composition [wt.%] | | | | | | 14·O/C at. Ratio | 14.N/C at. ratio |
|---|---|---|---|---|---|---|---|---|
| | **C** | **O** | **N** | **Na** | **Cl** | **S** | | |
| **EK standard** | 58.2$\pm$ 1.1 | 41.1$\pm$ 1.1 | 0.2$\pm$ 0.1 | 0.3 $\pm$0 | 0 | 0.1 $\pm$0 | 7.4$\pm$0.2 | 0$\pm$0 |
| **DIMCARB** | 58.9$\pm$ 1.6 | 34.4$\pm$ 1.4 | 6.7$\pm$1 | 0 | 0 | 0 | 6.1$\pm$0.2 | 1.4$\pm$0.2 |
| **$NH_4OH$** | 57.1$\pm$ 2.9 | 37.7$\pm$ 1.8 | 5.1$\pm$ 2.2 | 0.1 $\pm$0 | 0 | 0 | 6.9$\pm$0.3 | 1.1$\pm$0.5 |
| **DES1 in $NH_4OH$** | 51.8$\pm$ 0.1 | 39.7$\pm$ 0.1 | 8$\pm$0.1 | 0 | 0.5 $\pm$0 | 0 | 8.1$\pm$0 | 1.9$\pm$0 |

Wang et al.[44] analysed the surface morphology of their crude and purified EK extracted from pomegranate husk. They showed the crude extract was composed of larger irregular spheres, whereas the purified extract was in a form of smaller block-shaped structures. In our case, the EK standard showed spherical shapes of different sizes, which were all significantly larger compared to the structures in the $NH_4OH$ and DES1/$NH_4OH$ EK samples. Although the structures in the DIMCARB sample were relatively large, they seemed to readily break down to much smaller ones. This small particle size was likely the cause of some product loss during the washing procedure, since the small particles do not settle well during centrifugation compared to larger ones. Although most of the product was gathered in a pellet at the bottom of the centrifuge tube, we could still see a very fine mist in the supernatant (not shown) that we were not able to separate and, therefore, discarded it along with the supernatant. Although minimal, these losses might be a more considerable problem in large scale production. Therefore, more efficient separation techniques would have to be applied.

Considering the impurities, the EDS analysis showed the presence of N and Cl in the powdered EK samples. This could indicate the presence of some residual reaction media since DIMCARB, $NH_4OH$ and both DES contain N and the later also contains Cl. Therefore, downstream processes should involve removal of ammonia, if ellagic acid is specifically required.

*Kinetic modelling*

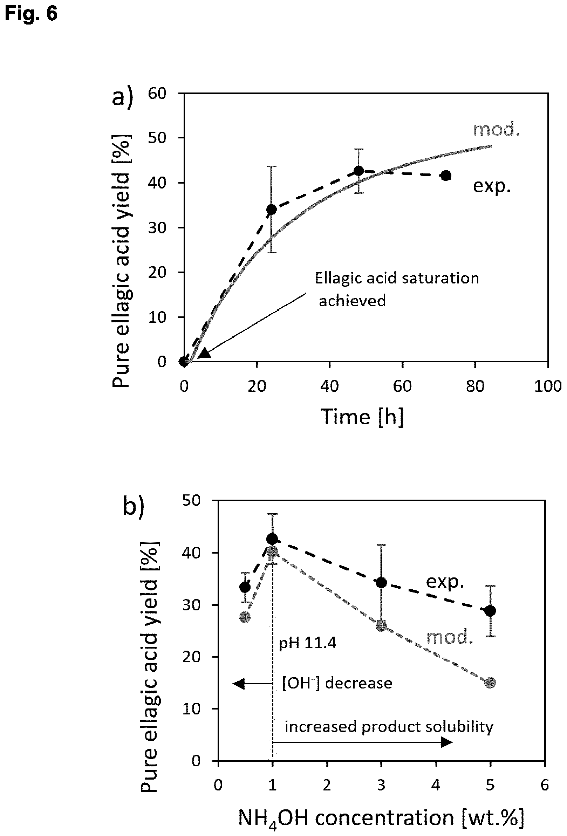

The model was initially based only on experiments with $NH_4OH$. As a weak base, it dissociates poorly, resulting in relatively small changes in pH at relevant reaction conditions when consumed. In contrast, KOH and NaOH dissociate almost completely, leading to large changes in pH and high sensitivity of the system, and were therefore not modelled. From the comparison between the modelling and experimental results of the yield of pure ellagic acid precipitate (Fig. 6), we can see that the trends can be described relatively well with only two reaction constants, where $k_f$ equals 46 $m^6/(kmol^2$ h) and $k_b$ equals 96 $m^3/(kmol$ h). Compared to the forward reaction constant of glycerol oleate-ethanol transesterification at 298 K (1.7 $m^6/(kmol^2$ h)), ellagic acid synthesis is relatively fast, although the upper limits of the reaction pH limit the overall reaction rate. In Fig. 6b we see the maximum yield of ellagic acid at 1 wt.% (pH 11.4), which is due to a low [OH -] at lower $NH_4OH$ concentrations and increased solubility at higher pH. The agreement could be improved by a more accurate solubility measurement, a pH measurement during the experiment and a determination of the by-products. In addition, we neglect here that the experimental product is the ammonium salt of ellagate, with about one nitrogen per molecule in the dried sample prepared with $NH_4OH$, as shown in the SEM-EDS analysis (Table 2**Fehler! Verweisquelle konnte nicht gefunden werden.**), resulting in a 5 % relative lower experimental yield.

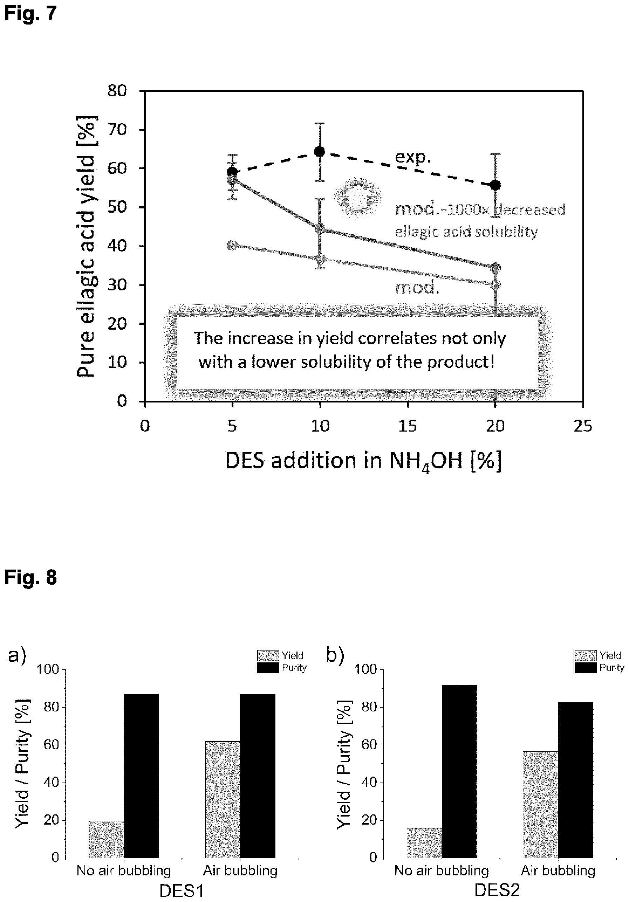

Moving to the DES1/$NH_4OH$ system, we can observe that pH slightly drops with DES addition (Table 1**Fehler! Verweisquelle konnte nicht gefunden werden.**), which was considered and is the only reason between the differences in simulations with DES amount variation. In the Fig. 7 we can observe predicted decrease of ellagic acid precipitate yield when DES amount was increased, correlating with pH decrease. From reference experiments with ellagic acid standard, we observed larger recovery of ellagic acid in the case of DES1/$NH_4OH$ than with $NH_4OH$ only (Fig. 18). Therefore, DES1 probably limits ellagic acid solubility and therefore increasing precipitate yield and limit backward reactions of ellagic acid. To test the implications to the extreme, we, in model, divided the ellagic acid solubility by 1000, which resulted in a significantly larger yield at low addition of DES1. However, at high DES1 addition, solubility modification does not have a large effect on the precipitate yield and is more closely correlated with pH. Therefore, some other factor relating to DES addition also influences the yield of precipitated product.

In the DES1/$NH_4OH$ system, we can observe that the pH drops slightly when DES is added (Table 1). This was considered and is the only reason for the differences in the simulations with DES amount variation. In Fig. 7 we can observe the predicted decrease in ellagic acid precipitation yield when the amount of DES was increased, which correlates with the pH decrease. In reference experiments with ellagic acid standard, we observed a larger yield of ellagic acid in the case of DES1/$NH_4OH$ than with $NH_4OH$ alone (Fig. 18). Therefore, DES1 probably limits the solubility of ellagic acid and thus increases the yield of the precipitate and limits the back reactions of ellagic acid. To push the effects to the extreme, we divided the solubility of ellagic acid by 1000 in the model, which resulted in a significantly higher yield with low addition of DES1. However, at high DES1 addition, the change in solubility does not have a large effect on the precipitate yield and is more closely correlated with pH. Therefore, another factor associated with the addition of DES also affects the yield of the precipitated product.

*Scale-up*

In order to test the potential feasibility of industrial production of EK, a simple scale-up process was also performed. We used only the reactions that yielded the best results, i.e. reactions with 10 % DES1 or DES2 in 1 % $NH_4OH$. The reactions were performed either with or without air bubbling. The results in Fig. 8a and b show that in the absence of air bubbling, the EK yields were only 19.7 % for DES1 and 15.8 % for DES2, compared to small scale reactions where the yields were 77.5 % for DES1 and 75.1 % for DES2. However, when air bubbling was introduced, the yields increased to 61.8 % and 56.6 %, respectively. The purity of all samples was between 82 and 92 %. Although the yields were not as good as in small-scale experiments, they were still relatively high and could probably be improved even further with some optimization like more efficient air bubbling (better dispersion throughout the reaction solution) or possibly the use of an additional oxidant. In addition, reactor with (semi) continuous pure solid EtGal addition and precipitate removal, pH and temperature control could be used. In this way, we could increase the product yield and decreased reactor downtown. However, the feasibility of a large-scale production is not dependent only on yields, but also on the process of product purification (including the potential toxicity of the impurities), which is not yet developed and therefore cannot be evaluated. Nonetheless, the results seem promising and can at least provide a starting point for further research and development.

**Conclusions**

In this study we attempted to synthetize EK from EtGal with the use of different catalysts, i.e. ionic liquid, hydroxides/aeration and DES/hydroxide/aeration. The highest yields were obtained in 10 % DES1 (ChCl:glycerol) in 1 % $NH_4OH$ and amounted to 77.5 % with 82.9 % purity. The impurities were most likely unspecific reaction products and intermediates, products of $EK-NH_4OH$ interaction and possibly some residual reaction media components. The process was efficient even at a larger scale (61.8 % yield). Using kinetic modelling, we were able to describe the trends in pure product yield with hydroxides using pH variation and ellagic acid solubility with only two kinetic parameters. Despite the observed reduction in ellagic acid solubility with DES1 (ChCl:glycerol), which we observed in a reference experiment and incorporated into the model, the kinetic modelling predicts that other DES-related factors also increase ellagic acid yield.

**References**

1. Vattem, D. A. & Shetty, K. Ellagic acid production and phenolic antioxidant activity in cranberry pomace (Vaccinium macrocarpon) mediated by Lentinus edodes using a solid-state system. Process Biochem. 39, 367-379 (2003).

2. Kannan, M. M. & Quine, S. D. Ellagic acid ameliorates isoproterenol induced oxidative stress: Evidence from electrocardiological, biochemical and histological study. Eur. J. Pharmacol. 659, 45-52 (2011).

3. Loarca-Piña, G., Kuzmicky, P. A., González De Mejía, E., Kado, N. Y. & Hsieh, D. P. M. Antimutagenicity of ellagic acid against aflatoxin B1 in the Salmonella microsuspension assay. *Mutat. Res. Mutagen. Relat. Subj.* **360,** 15-21 (1996).

4. Kelloff, G. J. et al. New agents for cancer chemoprevention. J. Cell. Biochem. 63, 1-28 (1996).

5. Losso, J. N., Bansode, R. R., Trappey, A., Bawadi, H. A. & Truax, R. In vitro antiproliferative activities of ellagic acid. J. Nutr. Biochem. 15, 672-678 (2004).

6. Umesalma, S. & Sudhandiran, G. Ellagic acid prevents rat colon carcinogenesis induced by 1, 2 dimethyl hydrazine through inhibition of AKT-phosphoinositide-3 kinase pathway. Eur. J. Pharmacol. 660, 249-258 (2011).

7. Rogerio, A. P. et al. Anti-inflammatory effects of Lafoensia pacari and ellagic acid in a murine model of asthma. Eur. J. Pharmacol. 580, 262-270 (2008).

8. Atta-Ur-Rahman et al. New antioxidant and antimicrobial ellagic acid derivatives from Pteleopsis hylodendron. Planta Med. 67, 335-339 (2001).

9. Khanbabaee, K. & van Ree, T. Tannins: Classification and Definition. Nat. Prod. Rep. 18, 641-649 (2001).

10. Zafrilla, P., Ferreres, F. & Tomás-Barberán, F. A. Effect of processing and storage on the antioxidant ellagic acid derivatives and flavonoids of red raspberry (Rubus idaeus) jams. J. Agric. Food Chem. 49, 3651-3655 (2001).

11. Haddock, E. A. et al. The metabolism of gallic acid and hexahydroxydiphenic acid in plants: Biogenetic and molecular taxonomic considerations. Phytochemistry 21, 1049-1062 (1982).

12. Bate-Smith, E. C. Detection and determination of ellagitannins. Phytochemistry 11, 1153-1156 (1972).

13. Khac, D. Do, Tran-Van, S., Campos, A. M., Lallemand, J. Y. & Fetizon, M. Ellagic compounds from Diplopanax stachyanthus. Phytochemistry 29, 251-256 (1990).

14. Jingjing, L. & Qipeng, Y. A new method for ellagic acid production from pomegranate husk. J. Food Process Eng. 31, 443-454 (2008).

15. Buenrostro-Figueroa, J. et al. Continuous production of ellagic acid in a packed-bed reactor. Process Biochem. 49, 1595-1600 (2014).

16. Sundaralingam, R., Premina, S. & Andrew, S. N. BIOCONVERSION OF ELLAGITANNINS TO ELLAGIC ACID FROM POMEGRANATE PEELS BY SOLID-STATE FERMENTATION USING ASPERGILLUS NIGER AND RHIZOPUS ORYZAE. PARIPEX INDIAN J. Res. 1-4 (2020) doi:10.36106/PARIPEX/1701247.

17. Zeng, W., Heur, Y. -H, Kinstle, T. H. & Stoner, G. D. Synthesis of [14CO]ellagic acid. J. Label. Compd. Radiopharm. 29, 657-666 (1991).

18. Chowdhury, S. A. et al. Efficient Synthesis of Ellagic Acid Salts Using Distillable Ionic Liquids. Aust. J. Chem. 64, 1624-1627 (2011).

19. Ren, Y. et al. Synthesis and AntitumorActivity of Ellagic Acid Peracetate. ACS Med. Chem. Lett. 3, 631 (2012).

20. Alam, A. & Tsuboi, S. Total synthesis of 3,3',4-tri-O-methylellagic acid from gallic acid. Tetrahedron 63, 10454-10465 (2007).

21. Alam, A., Takaguchi, Y. & Tsuboi, S. Synthesis of ellagic acid and its 4,4'-di-O-alky derivatives from gallic acid. (2005).

22. Daley, S. K. & Downer-Riley, N. The biomimetic synthesis of balsaminone A and ellagic acid via oxidative dimerization. Beilstein J. Org. Chem. 16, 2026 (2020).

23. Hayyan, A. et al. Encapsulated deep eutectic solvent for esterification of free fatty acid. Biomass Convers. Biorefinery 12, 3725-3735 (2022).

24. Gu, L., Huang, W., Tang, S., Tian, S. & Zhang, X. A novel deep eutectic solvent for biodiesel preparation using a homogeneous base catalyst. Chem. Eng. J. 259, 647-652 (2015).

25. Kadapure, S. A. et al. Optimization of conversion of Pongamia pinnata oil with high FFA to biodiesel using novel deep eutectic solvent. J. Environ. Chem. Eng. 5, 5331-5336 (2017).

26. Hayyan, A. et al. Reduction of high content of free fatty acid in sludge palm oil via acid catalyst for biodiesel production. Fuel Process. Technol. 92, 920-924 (2011).

27. Hayyan, A., Hashim, M. A., Hayyan, M., Mjalli, F. S. & Alnashef, I. M. A new processing route for cleaner production of biodiesel fuel using a choline chloride based deep eutectic solvent. J. Clean. Prod. 65, 246-251 (2014).

28. Likozar, B. & Levec, J. Transesterification of canola, palm, peanut, soybean and sunflower oil with methanol, ethanol, isopropanol, butanol and tert-butanol to biodiesel: Modelling of chemical equilibrium, reaction kinetics and mass transfer based on fatty acid composition. Appl. Energy 123, 108-120 (2014).

29. Grzybowski, M., Sadowski, B., Butenschön, H. & Gryko, D. T. Synthetic Applications of Oxidative Aromatic Coupling-From Biphenols to Nanographenes. Angew. Chemie - Int. Ed. 59, 2998-3027 (2020).

30. Eslami, A. C., Pasanphan, W., Wagner, B. A. & Buettner, G. R. Free radicals produced by the oxidation of gallic acid: An electron paramagnetic resonance study. Chem. Cent. J. 4, 1-4 (2010).

31. Prince, R. & Hospital, A. Copyright ( c) 1982 Pergamon Press Ltd . All rights reserved . CONTACT ACTIVATION : ELLAGIC ACID. (1982).

32. Hasegawa, M. et al. Deprotonation Processes of Ellagic Acid in Solution and Solid States. Monatshefte fur Chemie 134, 811-821 (2003).

33. Press, R. E. & Hardcastle, D. Some Physico-chemical Properties of Ellagic Acid. J. Appl. Chem. 19, 9-13 (1969).

34. Bala, I., Bhardwaj, V., Hariharan, S. & Kumar, M. N. V. R. Analytical methods for assay of ellagic acid and its solubility studies. J. Pharm. Biomed. Anal. 40, 206-210 (2006).

35. Hostnik, G., Tošović, J., Stumpf, S., Petek, A. & Bren, U. The influence of pH on UV/Vis spectra of gallic and ellagic acid: A combined experimental and computational study. *Spectrochim. Acta Part A Mol. Biomol. Spectrosc.* **267,** 120472 (2022).

36. Simić, A. Z. *et al.* Study of ellagic acid electro-oxidation mechanism. *Monatshefte fur Chemie* **144,** 121-128 (2013).

37. Abbott, A. P., Boothby, D., Capper, G., Davies, D. L. & Rasheed, R. K. Deep Eutectic Solvents formed between choline chloride and carboxylic acids: Versatile alternatives to ionic liquids. J. Am. Chem. Soc. 126, 9142-9147 (2004).

38. Dai, Y., van Spronsen, J., Witkamp, G. J., Verpoorte, R. & Choi, Y. H. Natural deep eutectic solvents as new potential media for green technology. Anal. Chim. Acta 766, 61-68 (2013).

39. Choi, Y. H. et al. Are natural deep eutectic solvents the missing link in understanding cellular metabolism and physiology? Plant Physiol. 156, 1701-1705 (2011).

40. Delorme, A. E., Andanson, J. M. & Verney, V. Improving laccase thermostability with aqueous natural deep eutectic solvents. Int. J. Biol. Macromol. 163, 919-926 (2020).

41. Kim, J. B. & Paik, Y. S. Stability of carthamin from Carthamus tinctorius in aqueous solution:pH and temperature effects. Arch. Pharm. Res. 20, 643-646 (1997).

42. Liu, W. & Wang, F. p-Toluenesulfonic Acid-based Deep Eutectic Solvent as Transesterification Catalyst for Biodiesel Production. J. Oleo Sci. 67, 1163-1169 (2018).

43. Lee, Y. R., Lee, Y. J., Ma, W. & Row, K. H. Determination of deep eutectic solvents as eco-friendly catalysts for biodiesel esterification from an alcohol-palmitic acid mixture. Korean J. Chem. Eng. 33, 2337-2341 (2016).

44. Wang, L. et al. Optimization of Ellagic Acid Purification from Pomegranate Husk by Antisolvent Recrystallization. Chem. Eng. Technol. 41, 1188-1198 (2018).

## Claims

1. A method for preparing ellagic acid directly from ethyl gallate, comprising the steps

   (i) reacting ethyl gallate with a hydroxide compound in the presence of air and a deep eutectic solvent (DES) at alkaline pH, and
   (ii) isolating ellagic acid.

2. The method according to claim 1, wherein the hydroxide compound is selected from ammonium hydroxide $NH_4OH$, alkali metal hydroxides such as NaOH and KOH, and mixtures thereof, preferably wherein the hydroxide compound is

NH$_4$OH.

3. The method according to claim 1 or 2, wherein the concentration of the hydroxide compound, in particular NH$_4$OH, is in a range of 0.5-5 % based on the total weight of the reaction mixture, preferably wherein the concentration of the hydroxide compound is in a range of 0.8-1.2 %, more preferably about 1 %.

4. The method according to any one of claims 1-3, wherein the pH is in a range of 9-13, preferably 10-12, more preferably about 11.

5. The method according to any one of claims 1-4, wherein the DES has an alkaline pH.

6. The method according to any one of claims 1-5, wherein the DES comprises 5-choline chloride (ChCl).

7. The method according to claim 6, wherein the DES comprises ChCl and at least one of glycerol and urea, preferably in a ratio of about 1:1 to 1:3, more preferably about 1:2 of ChCl:glycerol or ChCl:urea, respectively.

8. The method according to any one of claims 1-7, wherein the concentration of the DES is in a range of 5-20 % based on the total weight of the reaction mixture, preferably wherein the concentration of the DES is in a range of 8-12 %, more preferably about 10%.

9. The method according to any one of claims 1-8, comprising a step of preparing a solution comprising the hydroxide compound and the DES and adding ethyl gallate to the solution to yield a reaction mixture at alkaline pH, and subjecting the reaction mixture to the reaction in step (i).

10. The method according to any one of claims 1-9, wherein the reaction time in step (i) is about 24-72 h, preferably 42-54 h, more preferably about 48 h.

11. The method according to any one of claims 1-10, wherein the reaction in step (i) is carried out at room temperature.

12. The method according to any one of claims 1-11, wherein in step (i), the presence of air is provided by air bubbling.

13. The method according to any one of claims 1-12, wherein step (i) is carried out with stirring.

**Fig. 1**

**Fig. 2**

**Fig. 3**

**Fig. 4**

**Fig. 5**

**Fig. 6**

**Fig. 7**

**Fig. 8**

**Fig. 9**

**Fig. 10**

**Fig. 11**

## Fig. 12

## Fig. 13

## Fig. 14

## Fig. 15

## Fig. 16

**Fig. 17**

**Fig. 18**

**Fig. 19**

**Fig. 20**

**Fig. 21**

Europäisches
Patentamt

European
Patent Office

Office européen
des brevets

# EUROPEAN SEARCH REPORT

**Application Number**

EP 24 19 9711

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | CHOWDHURY SHAHANA A. ET AL: "Efficient Synthesis of Ellagic Acid Salts Using Distillable Ionic Liquids", AUSTRALIAN JOURNAL OF CHEMISTRY, vol. 64, no. 12, 1 January 2011 (2011-01-01), page 1624, XP093146847, AU ISSN: 0004-9425, DOI: 10.1071/CH11236 * the whole document * | 1-13 | INV. C07D493/06 |
| A | EP 0 390 107 A2 (KIKKOMAN CORP [JP]) 3 October 1990 (1990-10-03) * claims 1-8 * | 1-13 | |
| A | LEE YU RI ET AL: "Determination of deep eutectic solvents as eco-friendly catalysts for biodiesel esterification from an alcohol-palmitic acid mixture", KOREAN JOURNAL OF CHEMICAL ENGINEERING, SPRINGER US, NEW YORK, vol. 33, no. 8, 16 May 2016 (2016-05-16), pages 2337-2341, XP036023048, ISSN: 0256-1115, DOI: 10.1007/S11814-016-0073-Y [retrieved on 2016-05-16] * abstract * | 1-13 | |

TECHNICAL FIELDS SEARCHED (IPC)

C07D

-/--

The present search report has been drawn up for all claims

| Place of search | Date of completion of the search | Examiner |
|---|---|---|
| Munich | 9 January 2025 | Grégoire, Ariane |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

| | Europäisches Patentamt European Patent Office Office européen des brevets | **EUROPEAN SEARCH REPORT** | Application Number EP 24 19 9711 |

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | AN JUAN-YAN ET AL: "An efficiency strategy for extraction and recovery of ellagic acid from waste chestnut shell and its biological activity evaluation", MICROCHEMICAL JOURNAL, NEW YORK, NY, US, vol. 160, 9 October 2020 (2020-10-09), XP086388715, ISSN: 0026-265X, DOI: 10.1016/J.MICROC.2020.105616 [retrieved on 2020-10-09] * abstract * | 1-13 | |
| A,D | ANDREW P. ABBOTT ET AL: "Deep Eutectic Solvents Formed between Choline Chloride and Carboxylic Acids: Versatile Alternatives to Ionic Liquids", JOURNAL OF THE AMERICAN CHEMICAL SOCIETY, vol. 126, no. 29, 1 July 2004 (2004-07-01), pages 9142-9147, XP055254619, ISSN: 0002-7863, DOI: 10.1021/ja048266j * abstract * | 1-13 | |

TECHNICAL FIELDS SEARCHED (IPC)

The present search report has been drawn up for all claims

| Place of search | Date of completion of the search | Examiner |
|---|---|---|
| Munich | 9 January 2025 | Grégoire, Ariane |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 19 9711

This annex lists the patent family members relating to the patent documents cited in the above-mentioned European search report.
The members are as contained in the European Patent Office EDP file on
The European Patent Office is in no way liable for these particulars which are merely given for the purpose of information.

09-01-2025

| Patent document cited in search report | | Publication date | Patent family member(s) | | Publication date |
|---|---|---|---|---|---|
| EP 0390107 | A2 | 03-10-1990 | DE | 69021239 T2 | 25-01-1996 |
| | | | EP | 0390107 A2 | 03-10-1990 |
| | | | JP | H0791302 B2 | 04-10-1995 |
| | | | JP | H02255686 A | 16-10-1990 |

For more details about this annex : see Official Journal of the European Patent Office, No. 12/82

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **VATTEM, D. A.** ; **SHETTY, K.** Ellagic acid production and phenolic antioxidant activity in cranberry pomace (Vaccinium macrocarpon) mediated by Lentinus edodes using a solid-state system. *Process Biochem.*, 2003, vol. 39, 367-379
- **KANNAN, M. M.** ; **QUINE, S. D.** Ellagic acid ameliorates isoproterenol induced oxidative stress: Evidence from electrocardiological, biochemical and histological study. *Eur. J. Pharmacol.*, 2011, vol. 659, 45-52
- **KELLOFF, G. J. et al.** New agents for cancer chemoprevention. *J. Cell. Biochem.*, 1996, vol. 63, 1-28
- **LOSSO, J. N.** ; **BANSODE, R. R.** ; **TRAPPEY, A.** ; **BAWADI, H. A.** ; **TRUAX, R.** In vitro antiproliferative activities of ellagic acid. *J. Nutr. Biochem.*, 2004, vol. 15, 672-678
- **UMESALMA, S.** ; **SUDHANDIRAN, G.** Ellagic acid prevents rat colon carcinogenesis induced by 1, 2 dimethyl hydrazine through inhibition of AKT-phosphoinositide-3 kinase pathway. *Eur. J. Pharmacol.*, 2011, vol. 660, 249-258
- **ROGERIO, A. P. et al.** Anti-inflammatory effects of Lafoensia pacari and ellagic acid in a murine model of asthma. *Eur. J. Pharmacol.*, 2008, vol. 580, 262-270
- **ATTA-UR-RAHMAN et al.** New antioxidant and antimicrobial ellagic acid derivatives from Pteleopsis hylodendron. *Planta Med.*, 2001, vol. 67, 335-339
- **KHANBABAEE, K.** ; **VAN REE, T.** Tannins: Classification and Definition. *Nat. Prod. Rep.*, 2001, vol. 18, 641-649
- **ZAFRILLA, P.** ; **FERRERES, F.** ; **TOMÁS-BARBERÁN, F.** A. Effect of processing and storage on the antioxidant ellagic acid derivatives and flavonoids of red raspberry (Rubus idaeus) jams. *J. Agric. Food Chem.*, 2001, vol. 49, 3651-3655
- **HADDOCK, E. A. et al.** The metabolism of gallic acid and hexahydroxydiphenic acid in plants: Biogenetic and molecular taxonomic considerations. *Phytochemistry*, 1982, vol. 21, 1049-1062
- **BATE-SMITH, E. C.** Detection and determination of ellagitannins. *Phytochemistry*, 1972, vol. 11, 1153-1156
- **KHAC, D. DO** ; **TRAN-VAN, S.** ; **CAMPOS, A. M.** ; **LALLEMAND, J. Y.** ; **FETIZON, M.** Ellagic compounds from Diplopanax stachyanthus. *Phytochemistry*, 1990, vol. 29, 251-256

- **JINGJING, L.** ; **QIPENG, Y.** A new method for ellagic acid production from pomegranate husk. *J. Food Process Eng.*, 2008, vol. 31, 443-454
- **BUENROSTRO-FIGUEROA, J. et al.** Continuous production of ellagic acid in a packed-bed reactor. *Process Biochem.*, 2014, vol. 49, 1595-1600
- **SUNDARALINGAM, R.** ; **PREMINA, S.** ; **ANDREW, S. N.** BIOCONVERSION OF ELLAGITANNINS TO ELLAGIC ACID FROM POMEGRANATE PEELS BY SOLID-STATE FERMENTATION USING ASPERGILLUS NIGER AND RHIZOPUS ORYZAE. *PARIPEX INDIAN J. Res.*, 2020, 1-4
- **ZENG, W.** ; **HEUR, Y. -H** ; **KINSTLE, T. H.** ; **STONER, G. D.** Synthesis of [14CO]ellagic acid. *J. Label. Compd. Radiopharm.*, 1991, vol. 29, 657-666
- **CHOWDHURY, S. A. et al.** Efficient Synthesis of Ellagic Acid Salts Using Distillable Ionic Liquids. *Aust. J. Chem.*, 2011, vol. 64, 1624-1627
- **REN, Y. et al.** Synthesis and AntitumorActivity of Ellagic Acid Peracetate. *ACS Med. Chem. Lett.*, 2012, vol. 3, 631
- **ALAM, A.** ; **TSUBOI, S.** Total synthesis of 3,3',4-tri-O-methyllellagic acid from gallic acid. *Tetrahedron*, 2007, vol. 63, 10454-10465
- **ALAM, A.** ; **TAKAGUCHI, Y.** ; **TSUBOI, S.** *Synthesis of ellagic acid and its 4,4'-di-O-alky derivatives from gallic acid*, 2005
- **DALEY, S. K.** ; **DOWNER-RILEY, N.** The biomimetic synthesis of balsaminone A and ellagic acid via oxidative dimerization. *Beilstein J. Org. Chem.*, 2020, vol. 16, 2026
- **HAYYAN, A. et al.** Encapsulated deep eutectic solvent for esterification of free fatty acid. *Biomass Convers. Biorefinery*, 2022, vol. 12, 3725-3735
- **GU, L.** ; **HUANG, W.** ; **TANG, S.** ; **TIAN, S.** ; **ZHANG, X.** A novel deep eutectic solvent for biodiesel preparation using a homogeneous base catalyst. *Chem. Eng. J.*, 2015, vol. 259, 647-652
- **KADAPURE, S. A. et al.** Optimization of conversion of Pongamia pinnata oil with high FFA to biodiesel using novel deep eutectic solvent. *J. Environ. Chem. Eng.*, 2017, vol. 5, 5331-5336
- **HAYYAN, A. et al.** Reduction of high content of free fatty acid in sludge palm oil via acid catalyst for biodiesel production. *Fuel Process. Technol.*, 2011, vol. 92, 920-924

- **HAYYAN, A.** ; **HASHIM, M. A.** ; **HAYYAN, M.** ; **MJALLI, F. S.** ; **ALNASHEF, I. M.** A new processing route for cleaner production of biodiesel fuel using a choline chloride based deep eutectic solvent. *J. Clean. Prod.*, 2014, vol. 65, 246-251

- **LIKOZAR, B.** ; **LEVEE, J.** Transesterification of canola, palm, peanut, soybean and sunflower oil with methanol, ethanol, isopropanol, butanol and tert-butanol to biodiesel: Modelling of chemical equilibrium, reaction kinetics and mass transfer based on fatty acid composition. *Appl. Energy*, 2014, vol. 123, 108-120

- **GRZYBOWSKI, M.** ; **SADOWSKI, B.** ; **BUTENSCHÖN, H.** ; **GRYKO, D. T.** Synthetic Applications of Oxidative Aromatic Coupling-From Biphenols to Nanographenes. *Angew. Chemie - Int. Ed.*, 2020, vol. 59, 2998-3027

- **ESLAMI, A. C.** ; **PASANPHAN, W.** ; **WAGNER, B. A.** ; **BUETTNER, G. R.** Free radicals produced by the oxidation of gallic acid: An electron paramagnetic resonance study. *Chem. Cent. J*, 2010, vol. 4, 1-4

- **PRINCE, R.** CONTACT ACTIVATION : ELLAGIC ACID. Pergamon Press Ltd, 1982

- **HASEGAWA, M. et al.** Deprotonation Processes of Ellagic Acid in Solution and Solid States. *Monatshefte fur Chemie*, 2003, vol. 134, 811-821

- **PRESS, R. E.** ; **HARDCASTLE, D.** Some Physico-chemical Properties of Ellagic Acid. *J. Appl. Chem.*, 1969, vol. 19, 9-13

- **BALA, I.** ; **BHARDWAJ, V.** ; **HARIHARAN, S.** ; **KUMAR, M. N. V. R.** Analytical methods for assay of ellagic acid and its solubility studies. *J. Pharm. Biomed. Anal.*, 2006, vol. 40, 206-210

- **ABBOTT, A. P.** ; **BOOTHBY, D.** ; **CAPPER, G.** ; **DAVIES, D. L.** ; **RASHEED, R. K.** Deep Eutectic Solvents formed between choline chloride and carboxylic acids: Versatile alternatives to ionic liquids. *J. Am. Chem. Soc.*, 2004, vol. 126, 9142-9147

- **DAI, Y.** ; **VAN SPRONSEN, J.** ; **WITKAMP, G. J.** ; **VERPOORTE, R.** ; **CHOI, Y. H.** Natural deep eutectic solvents as new potential media for green technology. *Anal. Chim. Acta*, 2013, vol. 766, 61-68

- **CHOI, Y. H. et al.** Are natural deep eutectic solvents the missing link in understanding cellular metabolism and physiology?. *Plant Physiol.*, 2011, vol. 156, 1701-1705

- **DELORME, A. E.** ; **ANDANSON, J. M.** ; **VERNEY, V.** Improving laccase thermostability with aqueous natural deep eutectic solvents. *Int. J. Biol. Macromol.*, 2020, vol. 163, 919-926

- **KIM, J. B.** ; **PAIK, Y. S.** Stability of carthamin from Carthamus tinctorius in aqueous solution:pH and temperature effects. *Arch. Pharm. Res.*, 1997, vol. 20, 643-646

- **LIU, W.** ; **WANG, F.** p-Toluenesulfonic Acid-based Deep Eutectic Solvent as Transesterification Catalyst for Biodiesel Production. *J. Oleo Sci.*, 2018, vol. 67, 1163-1169

- **LEE, Y. R.** ; **LEE, Y. J.** ; **MA, W.** ; **ROW, K. H.** Determination of deep eutectic solvents as eco-friendly catalysts for biodiesel esterification from an alcohol-palmitic acid mixture. *Korean J. Chem. Eng.*, 2016, vol. 33, 2337-2341

- **WANG, L. et al.** Optimization of Ellagic Acid Purification from Pomegranate Husk by Antisolvent Recrystallization. *Chem. Eng. Technol.*, 2018, vol. 41, 1188-1198